# EUROPEAN PATENT APPLICATION

(11) **EP 4 600 237 A1**
(43) Date of publication of application: **13.08.2025**
(21) Application number: 24872699.4
(22) Date of filing: 30.07.2024
(51) Int. Cl.: C07C 2/08, C07C 7/00, C07C 7/09, C07C 11/107

(54) **METHOD FOR PREPARING OLIGOMER**

(30) Priority: 26.09.2023 KR 20230128683; 29.07.2024 KR 20240099986
(71) Applicant: LG CHEM, LTD., Seoul 07336 (KR)
(72) Inventor: SONG, Jong Hun, Daejeon 34122 (KR); HWANG, Moon Sub, Daejeon 34122 (KR); LEE, Jeong Seok, Daejeon 34122 (KR); PAK, Geun Tae, Daejeon 34122 (KR)
(74) Representative: Hoffmann Eitle
(86) International application number: PCT/KR2024/011084
(87) International publication number: WO 2025/070990

(57) **Abstract**

Provided is a method of preparing an oligomer including: (S1) supplying an ethylene gas to a lower liquid area including a solvent in a reactor to perform an oligomerization reaction, discharging an unreacted ethylene gas to an upper portion of the reactor, and discharging a liquid stream including an oligomer product to a lower side of the reactor; (S2) supplying the liquid stream discharged from the lower side of the reactor to a separation column, and separating gas from the liquid stream to obtain a stream including the oligomer product; and (S3) supplying the gas discharged from the upper portion of the reactor to a compressor in which a recirculation pipe is installed to compress the gas, and then recirculating the compressed gas to the reactor, wherein a part of the compressed gas discharged from the compressor diverges to the compressor, and the compressed gas which is diverged is recirculated to a supply line of the compressor through a pressure regulating valve provided in the recirculation pipe of the compressor to control pressure in the supply line of the compressor.

## Description

### [Technical Field]

### CROSS-REFERENCE TO RELATED APPLICATIONS

The present application claims the benefit of priorities to Korean Patent Application No. 10-2023-0128683 filed on September 26, 2023 and Korean Patent Application No. 10-2024-0099986 filed on July 29, 2024, the entire contents of which are incorporated herein as a part of the specification.

### Technical Field

The present invention relates to a method of preparing an oligomer, and more particularly, to a method of preparing an oligomer which may improve catalyst reaction and process operation safety by maintaining constant pressure in a reactor in a process of recirculating unreacted gas.

### [Background Art]

An α-olefin (alpha-olefin) is an important material which is used in comonomers, cleaning agents, lubricants, plasticizers, and the like and is commercially widely used, and in particular, 1-hexene and 1-octene are often used as a comonomer for adjusting the density of polyethylene in the preparation of linear low-density polyethylene (LLDPE).

The α-olefin may be prepared by performing an oligomerization reaction in the state in which ethylene is dissolved in a solvent in the presence of a catalyst, and the oligomerization reaction may be performed in, for example, a bubble column reactor.

In the bubble column reactor, a raw gas is introduced to a liquid area including a solvent, a catalyst, and the like through a distribution device installed in the lower portion and is dispersed while rising in a bubble form, and the dispersed gas is mixed in the liquid area while producing turbulence, thereby performing the oligomerization reaction.

After the oligomerization reaction is performed, by-products, a solvent, unreacted gas dissolved in the solvent, and the like are included with an oligomer product in which ethylene is polymerized, in a stream in the liquid area in the reactor. Therefore, the discharge stream of the liquid area is supplied to a separation column to separate the unreacted gas, the solvent, and the like, thereby obtaining a refined oligomer product.

Meanwhile, after a significant amount of unreacted gas among the raw gas introduced to the bubble column reactor is discharged to the upper portion of the reactor, it may be supplied to the reactor again and used as a raw gas.

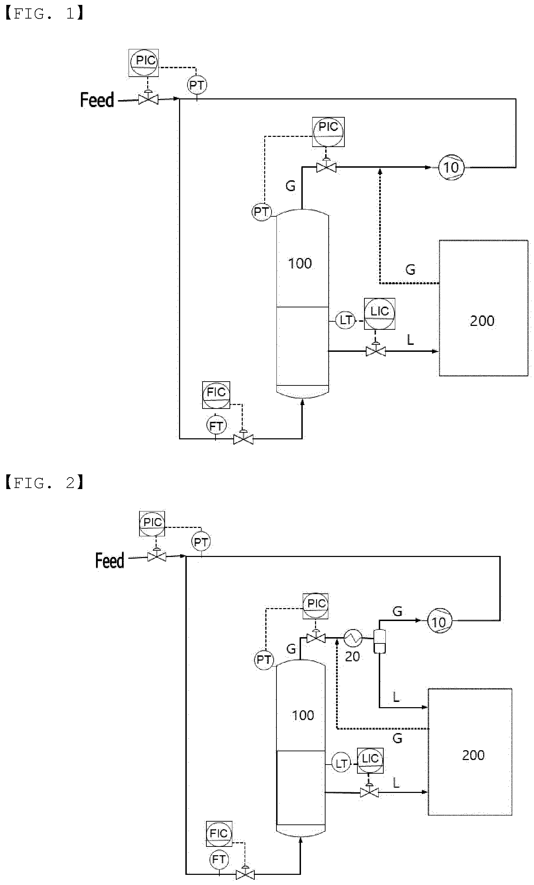

FIG. 1 shows a recyle loop of unreacted gas applied to a conventional oligomer preparation process, and the unreacted gas discharged from the upper portion of a reactor 100 may be recirculated to the reactor through a compressor 10. In addition, gas recovered in a separation column 200 of an oligomer product may also be transferred to a supply line of the compressor 10 and recirculated again to the reactor through a recirculation process. Herein, when a design flow rate of the compressor 10 and a flow rate of the unreacted gas discharged from the reactor 100 are different, pressure fluctuation may occur in the supply line of the compressor. In addition, even when the flow rates of gas recovered in a separation column 200 are combined in the supply line of the compressor, the pressure fluctuation may occur.

When the pressure fluctuation occurs in the supply line of the compressor 10 to which the unreacted gas discharged in the reactor 100 is transferred, the differential pressure of a pressure regulating valve provided in the reactor in a front stage is affected so that it is difficult to maintain constant internal pressure, and pressure fluctuation occurs also in a discharge line of the compressor, and thus, flow rate hunting of recirculating gas supplied to the reactor may be increased. The pressure fluctuation in the reactor and the flow rate hunting of gas supply change a liquid level in the reactor, resulting in a change in a catalyst residence time, and thus, reaction productivity is affected by activity of a catalyst reaction and a selectivity change and heat dissipating operability may be deteriorated by a change in a heating value.

In addition, low-boiling point products and a reaction solvent may be included in unreacted gas for reactor recirculation. As shown in FIG. 2, a process of installing a condenser 20 between a reactor and a compressor and supplying unreacted gas to the condenser to separate a liquid stream in which low-boiling point products and a reaction solvent are condensed and a gaseous stream of uncondensed and unreacted gas may be added. Herein, when the pressure fluctuation occurs in the supply line of the compressor, the amount and the composition of the condensate obtained in the condenser may be changed, and the concentration of the low-boiling point product in gas which is re-added to the reactor through the compressor is changed, which may affect reactivity in the reactor.

However, there is still no special device for preventing pressure fluctuation in the supply line of the compressor occurring in a process of recirculating unreacted gas to a reactor, and thus, there is a limitation in that pressure in the reactor fluctuates or gas recovered in the separation column is not stably added to the supply line of the compressor.

### [Disclosure]

### [Technical Problem]

In order to solve the problems mentioned in the Background Art, an object of the present invention is to provide a method of preparing an oligomer which may improve catalyst reaction and process operation safety by controlling pressure between a reactor and a compressor in a process of recirculating unreacted gas to a reactor to maintain constant pressure in the reactor.

### [Technical Solution]

In one general aspect, a method of preparing an oligomer includes: (S1) supplying an ethylene gas to a lower liquid area including a solvent in a reactor to perform an oligomerization reaction, discharging an unreacted ethylene gas to an upper portion of the reactor, and discharging a liquid stream including an oligomer product to a lower side of the reactor; (S2) supplying the liquid stream discharged from the lower side of the reactor to a separation column, and separating gas from the liquid stream to obtain a stream including the oligomer product; and (S3) supplying the gas discharged from the upper portion of the reactor to a compressor in which a recirculation pipe is installed to compress the gas, and then recirculating the compressed gas to the reactor, wherein a part of the compressed gas discharged from the compressor diverges to the compressor, and the compressed gas which is diverged is recirculated to a supply line of the compressor through a pressure regulating valve provided in the recirculation pipe of the compressor to control pressure in the supply line of the compressor.

In the present invention, the pressure regulating valve provided in the recirculation pipe of the compressor may remain open to continuously recirculate the compressed gas which is diverged.

### [Advantageous Effects]

According to the present invention, in an oligomer preparation process, an unreacted ethylene gas is compressed in a compressor, and then a part of the compressed gas discharged from the compressor diverges and is recirculated to a supply line of the compressor, thereby compensating for pressure fluctuation in the supply line of the compressor even when a flow rate of unreacted gas discharged from the reactor is lower than a designed flow rate of the compressor. In particular, the pressure regulating valve provided in the recirculation pipe of the compressor is continuously opened by an appropriate size design appropriate for the pressure fluctuation compensation, thereby properly adjusting the flow rate of the compressed gas recirculated to the supply line of the compressor to maintain constant pressure in the supply line of the compressor even when a change in a flow rate of the unreacted gas discharged from the reactor occurs.

Thus, pressure fluctuation between the reactor and the compressor in a process of recirculating an unreacted ethylene gas to a reactor may be prevented, and thus, the flow rate hunting of recirculating gas supplied to the reactor is minimized while also the internal pressure in the reactor is maintained constant, thereby maintaining constant liquid level and catalyst reaction in the reactor to improve the catalyst reaction for oligomer production and operability of process safety.

### [Description of Drawings]

FIGS. 1 and 2 show recyle loops of unreacted gas to a reactor, which is applied to a conventional olefin preparation process.
FIGS. 3 to 6 show recyle loops of unreacted gas to a reactor, including a structure in which a recirculation pipe is installed in a supply line and a discharge line of a compressor in an olefin preparation process according to an exemplary embodiment of the present invention.

### [Best Mode]

The terms and words used in the description and claims of the present invention are not to be construed limitedly as having general or dictionary meanings but are to be construed as having meanings and concepts meeting the technical ideas of the present invention, based on a principle that the inventors are able to appropriately define the concepts of terms in order to describe their own inventions in the best mode.

The meaning of "comprising" or "containing" used herein embodies specific characteristics, domains, integers, steps, actions, elements, or components, and does not exclude the addition of other specific characteristics, domains, integers, steps, actions, elements, or components.

The term "block copolymer" used in the present application refers to two or more monomer-derived repeating units being composed of a block form. The "block copolymer" may include a di-block copolymer, a tri-block copolymer, or a multiblock copolymer.

The term "stream" used in the present application may refer to a fluid flow in a process, or may refer to a fluid itself flowing in a pipe. Specifically, the stream may refer to both a fluid itself flowing in a pipe connecting each device and a fluid flow. In addition, the fluid may include any one or more components of gas, liquid, and solid.

The term "upper portion" used herein refers to, unless otherwise stated, a point of a height from 0 to 50 % from the top to the bottom of an apparatus, and specifically, may refer to a top (column top). In addition, the term "lower" refers to a point of a height from 50 to 100% from the top to the bottom of an apparatus, and specifically, may refer to a bottom (column bottom).

The term "side stream" used herein refers to, unless otherwise stated, a stream discharged from a height from 25 to 80% or discharged from a height from 40 to 70% from the top to the bottom of an apparatus.

In addition, "pressure" mentioned herein refers to gauge pressure measured based on atmospheric pressure.

Hereinafter, the present invention will be described in detail with reference to the attached drawings.

An exemplary embodiment of the present invention relates to a method of preparing an oligomer which may improve a catalyst reaction and process operation stability by maintaining constant reactor pressure in a process of recirculating unreacted gas to a reactor.

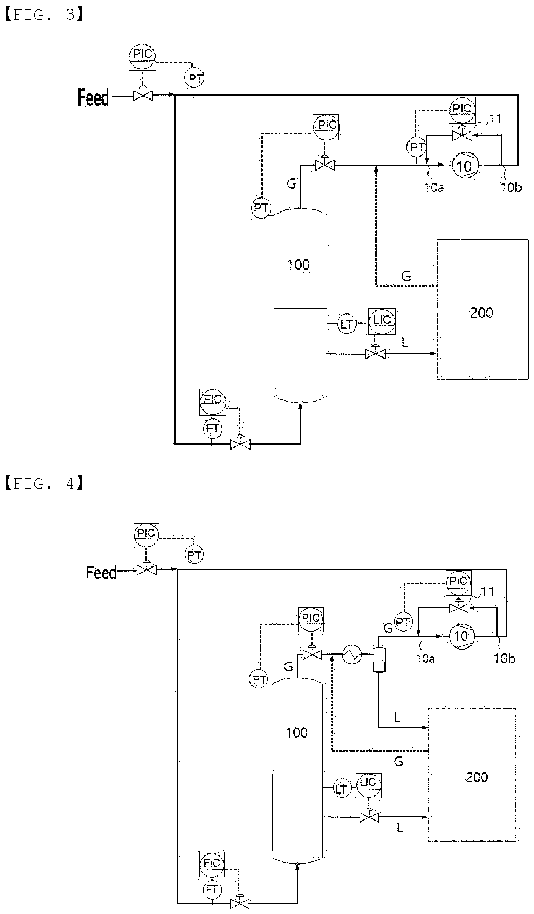

FIGS. 3 to 6 illustrate processes of a method of preparing an olefin according to an exemplary embodiment of the present invention, and the method may be performed in a system provided with a recyle loop of unreacted gas including: a reactor 100, a separation column 200, and a compressor 10 in which a recirculation pipe is installed. In addition, in the separation column, a reboiler for heating by heat transfer, a condenser which converts a stream produced by the heating from a gas phase into a liquid phase, a gas-liquid separator for separating the condensed stream, a valve which controls each stream flow, a pump, a sensor which measures temperature and pressure, and the like may be further installed.

According to an exemplary embodiment of the present invention, first, an ethylene gas is supplied to a lower liquid area including a solvent in a reactor 100 to perform an oligomerization reaction, and a catalyst for reaction activation may be further included in the liquid area.

The reactor 100 may be a reactor which may continuously perform an oligomerization reaction in a state in which ethylene is dissolved in a solvent in the presence of a catalyst, and the reactor may include a continuous stirred-tank reactor, a plug flow reactor, a bubble column reactor, and the like.

For example, a raw gas is introduced to a liquid area including a solvent, a catalyst, and the like through a distribution device installed in the lower portion of the bubble column reactor and is dispersed while rising in a bubble form, and the dispersed gas is mixed in the liquid area while producing turbulence, thereby performing the oligomerization reaction.

The oligomerization reaction may produce a target α-olefin product by supplying an ethylene monomer gas to the reactor 100. Herein, the oligomerization reaction is performed in a lower area of the reactor 100, and the oligomerization reaction of a monomer may be performed in a liquid state in which a gaseous ethylene monomer is dissolved in solvent.

The oligomerization reaction may refer to a reaction in which a monomer is oligomerized. The oligomerization may be referred to as trimerization or tetramerization depending on the number of monomers to be polymerized, and these are collectively called multimerization. For example, α-olefins such as 1-hexene and 1-octene may be prepared by a trimerization reaction or a tetramerization reaction of ethylene.

The ethylene gas supplied to the reactor 100 may be a stream including ethylene (C2) separated from naphtha cracking.

A solvent for dissolving the ethylene gas may include one or more selected from the group consisting of n-pentane, n-hexane, n-heptane, cyclohexane, methylcyclohexane, octane, cyclooctane, decane, dodecane, benzene, xylene, 1,3,5-trimethylbenzene, toluene, ethylbenzene, chlorobenzene, dichlorobenzene, and trichlorobenzene. The solvent may be used in combination of two or more, if necessary. Thus, the ethylene gas may be liquefied at a higher temperature and a dissolution rate at which the ethylene gas is dissolved in the solvent may be improved.

In the oligomerization reaction of ethylene, a compound including a transition metal-based catalyst may be used as a catalyst for promoting reaction activity. For example, the catalyst may be a compound including one or more selected from the group consisting of chromium (III) acetylacetonate, chromium (III) chloride tetrahydrofuran, chromium (III) 2-ethylhexanoate, chromium (III) tris(2,2,6,6-tetramethyl-3,5-heptanedionate), chromium (III) benzoylacetonate, chromium (III) hexafluoro-2,4-pentanedionate, chromium (III) acetate hydroxide, chromium (III) acetate, chromium (III) butyrate, chromium (III) pentanoate, chromium (III) laurate, and chromium (III) stearate.

In addition, a cocatalyst for increasing catalytic activity may be further used, and for example, the cocatalyst may include one or more selected from the group consisting of trimethyl aluminum, triethyl aluminum, triisopropyl aluminum, triisobutyl aluminum, ethylaluminum sesquichloride, diethylaluminum chloride, ethyl aluminum dichloride, methylaluminoxane, modified methylaluminoxane, and borate.

The oligomerization reaction may be operated under the conditions which are commonly applied in the art, and may be performed, for example, at a temperature of 30°C to 150°C or 50°C to 120°C and a pressure of 20 bar to 65 bar or 20 bar to 40 bar.

When the oligomerization reaction is performed, a liquid stream including an oligomer product and by-products produced by polymerization of ethylene, a solvent, an unreacted gas dissolved in the solvent, and the like may be present in the lower area, and an unreacted ethylene gas which is not dissolved in the solvent and does not participate in the oligomerization reaction may be present in the upper area, in the reactor 100.

The liquid stream may be discharged through a discharge line connected to a lower side of the reactor 100 and transferred to the separation column 200.

The separation column 200 may be supplied with the liquid stream, and perform component separation depending on a boiling point to separate a gaseous upper discharge stream including an unreacted ethylene monomer and a liquid lower discharge stream including an oligomer product, by-products, and a solvent. The oligomer product and the solvent included in the lower discharge stream of the separation column 200 may be supplied to an additional separation column (not shown) to separate the solvent therefrom, thereby obtaining a refined oligomer product. The separation column 200 and the additional separation column may be operated under commonly applied conditions in the art, and are not particularly limited.

Meanwhile, the unreacted ethylene gas which does not participate in the oligomerization reaction in the reactor 100 may be discharged to the upper portion of the reactor, and then recirculated to the reactor 100 through the compressor 10 in order to reuse the gas as a raw gas.

The compressor 10 supplies electric energy to the unreacted ethylene gas transferred from the reactor 100 to increase pressure by compression, and a temperature of the gaseous stream may be raised together in proportion to the amount of the electric energy.

As illustrated in FIGS. 3 to 6, in the present invention, a recirculation pipe provided with a pressure regulating valve 11 is connected to a supply line 10a of the compressor to which the unreacted gas is transferred and a discharge line 10b from which compressed gas passing through the compressor comes out, and a part of the compressed gas may diverge in the compressor discharge line 10b and be recirculated to prevent occurrence of pressure fluctuation in the supply line 10a of the compressor.

That is, a part of the compressed gas discharged from the compressor 10 may be used as a source for maintaining pressure in the supply line 10a of the compressor to compensate for the pressure fluctuation, even when the flow rate of the unreacted gas discharged from the reactor 100 is lower than the designed flow rate of the compressor 10. Thus, a change in differential pressure of the pressure regulating valve provided in the reactor in the front stage may be minimized to maintain constant pressure inside the reactor, and furthermore, prevent pressure fluctuation in the compressor discharge line 10b to minimize the flow rate hunting of recirculating gas supplied to the reactor 100. As a result, a liquid level and a catalyst reaction in the reactor are maintained constant, thereby improving oligomer productivity and process operation stability.

In addition, it is preferred that the pressure regulating valve 11 provided in the recirculation pipe of the compressor 10 maintains an open state. Since the pressure regulating valve provided in the recirculation pipe of a general compressor only controls pressure in the pipe from decreasing to a certain numerical value or less by a function of opening the pressure regulating valve only when a surge phenomenon in which gas is not introduced to the compressor occurs and closing the pressure regulating valve when normal operation is recovered, pressure hunting may occur in the supply line of the compressor. However, in the present invention, the capacity of the compressor is designed to be larger than the sum of the flow rate of gas discharged from the reactor and the flow rate of gas discharged from the separation column, and the pressure regulating valve 11 provided in the compressor recirculation pipe is designed to respond to the fluctuations of the flow rate of gas discharged from the reactor and the flow rate of gas discharged from the separation column to maintain constant openness, thereby adjusting the flow rate of the compressed gas which is diverged and maintaining constant pressure in the supply line 10a of the compressor.

In an exemplary embodiment of the present invention, various devices known in the art may be used without a limitation as long as the compressor 10 is a device capable of compressing gaseous flows, and one compressor or a plurality of compressors connected in series may be applied depending on a compression ratio of supplied unreacted gas.

Referring to FIG. 3, the unreacted ethylene gas recovered in the separation column 200 may be transferred to the supply line 10a of the compressor for recirculation to the reactor. Even when the flow rate of the unreacted gas discharged from the reactor and the flow rate of the gas discharged from the separation column are combined in the supply line 10a of the compressor to increase pressure fluctuation possibility, in the present invention, a part of the compressed gas discharged from the compressor diverges through the pressure regulating valve 11 provided in the recirculation pipe of the compressor 10 and is recirculated, so that the pressure in the supply line 10a of the compressor is controlled to minimize a change in differential pressure of the pressure regulating valve provided in the reactor in the front stage of the compressor, thereby maintaining constant pressure inside the reactor.

The capacity of the compressor 10 may be selected considering a total flow rate of the unreacted gas discharged from the reactor, the gas recovered in the separation column and the compressed gas recirculated in the compressor discharge line, which may be combined in the supply line 10a of the compressor.

Meanwhile, the flow rate of the compressed gas which is diverged from the compressor discharge line 10b and is recirculated through the pressure regulating valve 11 may be adjusted by measuring pressure in the supply line 10a of the compressor, and may be, for example, in a range of 5 to 50 wt%, specifically 10 to 30 wt% of the total flow rate of the compressed gas discharged from the compressor 10. When the flow rate of the compressed gas recirculated is less than 5 wt%, there is a limitation to compensating for pressure fluctuation occurring in the supply line 10a of the compressor, so that it is difficult to maintain constant pressure in the reactor 100. When the flow rate of the recirculated compressed gas is more than 50%, pressure fluctuation occurring in the supply line 10a of the compressor may be sufficiently controlled, but the amount of electricity consumed in the compressor 10 is increased, which is unfavorable in terms of energy.

Referring to FIG. 4, the unreacted gas discharged from the reactor and gas recovered in the separation column may pass through a condenser 20 placed in the front stage of the compressor 10 to condense low-boiling point components including an ethylene dimer and the solvent, and then a gaseous stream of the unreacted ethylene monomer from which a condensate is separated in the gas-liquid separator may be supplied to the compressor 10 for recirculation. Herein, a part of the compressed gas with elevated temperature after passing the compressor 10 is recirculated to the supply line 10a of the compressor at a constant amount range, for example, 5 to 50 wt% of the total flow rate of the compressed gas discharged from the compressor, thereby preventing the unreacted ethylene monomer stream discharged from the gas-liquid separator from condensing in the pipe depending on temperature conversion of outside air and the like. Thus, the condensate (liquid component) is prevented from being supplied to the compressor regardless of the composition of gas discharged through the condenser and the gas-liquid separator, so that recirculation of the unreacted gas to the reactor may be stably performed while protecting the pressure to maintain constant pressure in the reactor.

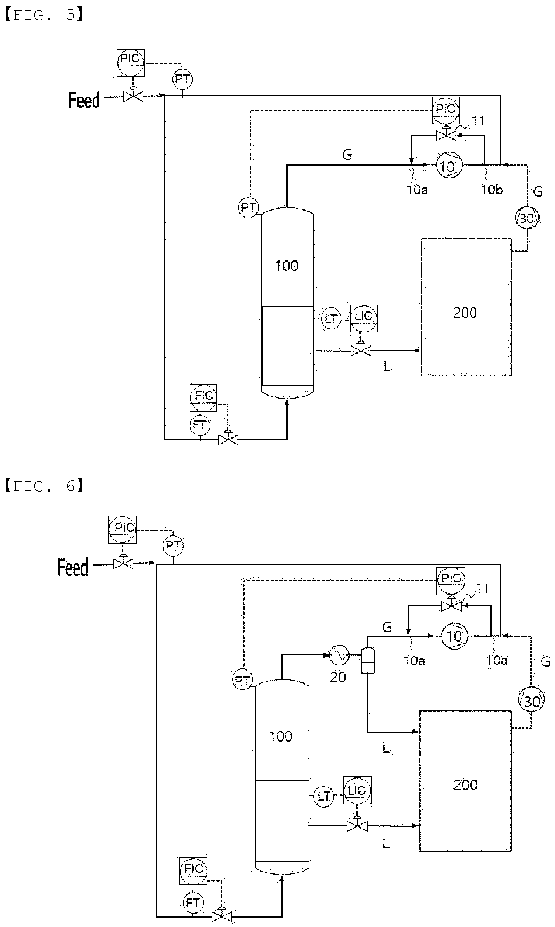

Referring to FIGS. 5 and **6****,** the unreacted ethylene gas recovered in the separation column 200 may be compressed in advance in a separate compressor 30, and then recirculated to the reactor 100 with the compressed gas discharged from the compressor 10. In this case also, in the present invention, a part of the compressed gas diverges through the pressure regulating valve 11 provided in the recirculation pipe of the compressor 10 and is recirculated, so that the pressure in the supply line 10a of the compressor, that is, in the upper portion of the reactor, is directly controlled to maintain constant pressure inside the reactor, and also, the unreacted gas discharged from the reactor is pressurized in the compressor and then recirculated to the lower portion of the reactor again.

According to the present invention as described above, in the oligomer preparation process, the unreacted ethylene gas is compressed in the compressor and then a part of the compressed gas discharged from the compressor diverges and is recirculated to the supply line of the compressor, thereby compensating for pressure fluctuation in the supply line of the compressor even when the flow rate of the unreacted gas discharged from the reactor is less than the designed flow rate of the compressor or the flow rate hunting of the unreacted gas discharged from the reactor occurs. In particular, the pressure regulating valve provided in the recirculation pipe of the compressor appropriately adjusts the flow rate of the compressed gas recirculating to the supply line of the compressor while being continuously opened, thereby maintaining constant pressure in the front stage, that is, the supply line of the compressor. For example, the pressure fluctuation in the supply line of the compressor may be controlled within a range of -1 bar to 1 bar, specifically -0.2 bar to 0.2 bar, or -0.1 bar to 0.1 bar.

As such, the pressure fluctuation between the reactor and the compressor in the process of recirculating the unreacted ethylene gas may be prevented, thereby maintaining constant pressure inside the reactor. For example, in the present invention, the pressure fluctuation inside the reactor operated in the oligomer preparation process may be controlled within a range of -0.5 bar to 0.5 bar, specifically -0.1 bar to 0.1 bar, and more specifically - 0.02 bar to 0.02 bar, so that stable operation of the reactor may be performed.

In addition, the flow rate hunting of the recirculating gas supplied to the reactor is minimized to maintain constant liquid level and catalyst reaction in the reactor, thereby improving oligomer productivity and process operation stability.

### [Mode for Carrying Out the Invention]

Hereinafter, the present invention will be described in more detail by the examples. However, the following examples are provided for illustrating the present invention, and it is apparent to a person skilled in the art that various modifications and alterations may be made without departing from the scope and spirit of the present invention, and the scope of the present invention is not limited thereto.

In the following examples and comparative examples, the method according to the present invention was simulated using a commercial process simulation program (HYSYS DANAMICS). Constants required for simulation used values embedded in the program, values described in the literatures, and the like.

### Comparative Example 1:

An oligomer preparation process was performed according to the process flow diagram shown in FIG. 1.

### (Step 1)

In the reactor 100, an ethylene gas was supplied to a lower liquid area including a solvent and a catalyst to perform an oligomerization reaction, unreacted ethylene gas (G) was discharged to the upper portion of the reactor 100, and a liquid stream (L) including an oligomer product was discharged to a lower side of the reactor.

### (Step 2)

A liquid stream (L) discharged from a lower side of the reactor 100 was supplied to a separation column 200, and gas was separated from the liquid stream to obtain a stream including an oligomer product.

### (Step 3)

Unreacted ethylene gas discharged from the upper portion of the reactor 100 was supplied to the compressor 10, and the capacity of the compressor was designed to be larger by 30% than the capacity of the supply line of the compressor. In addition, gas which was separated and recovered in the separation column 200 was transferred to the compressor 10 and compressed in the compressor 10 with the unreacted gas discharged from the reactor.

The compressed gas discharged from the compressor 10 was recirculated to the reactor 100 and used as a raw gas again.

### Comparative Example 2:

As shown in FIG. 2, the process was performed in the same manner as in Comparative Example 1, except that the unreacted gas discharged from the reactor and the unreacted gas discharged from the separation column were supplied to a condenser 20 to separate a liquid stream including low-boiling point components including an ethylene dimer and a condensed solvent and a gaseous stream including uncondensed unreacted gas, and then the uncondensed unreacted gas was supplied to the compressor 10 and the condensed liquid phase was transferred to a refining column 200 again.

### Example 1:

As shown in FIG. 3, an oligomer preparation process was performed according to a process flow diagram including a structure in which a recirculation pipe having a pressure regulating valve provided in the supply line and the discharge line of the compressor was installed.

### (Step 1)

An ethylene gas was supplied to a lower liquid area including a solvent and a catalyst in a reactor 100 to perform an oligomerization reaction, and then unreacted ethylene gas (G) was discharged to the upper portion of the reactor 100 and a liquid stream (L) including an oligomer product was discharged to a lower side of the reactor.

### (Step 2)

The liquid stream (L) discharged from a lower side of the reactor 100 was supplied to a separation column 200, and unreacted ethylene gas was separated and discharged from the liquid stream to obtain a stream including an oligomer product.

### (Step 3)

The unreacted ethylene gas discharged from the upper portion of the reactor 100 was supplied to the compressor 10, and at this time, gas separated and recovered in the separation column 200 was also transferred to the compressor 10 and compressed in the compressor 10 with the unreacted gas discharged from the reactor.

A part of the compressed gas was diverged in the compressor discharge line 10b and recirculated to a supply line 10a of the compressor, and at this time, the capacity of the compressor was designed so that the flow rate of the compressed gas which is diverged and recirculated in the compressor discharge line 10b was 5 wt% of the total compressed gas flow rate. In addition, the regulating valve provided in the compressor recirculation pipe was maintained in a constant open state to continuously perform recirculation of compressed gas which is diverged.

Meanwhile, the remaining compressed gas discharged from the compressor 10 was recirculated to the reactor 100 and used as raw gas again.

### Example 2:

As shown in FIG. 4, the process was performed in the same manner as in Example 1, except that the unreacted gas discharged from the reactor and gas recovered in the separation column were supplied to a condenser 20 to separate a liquid stream including low-boiling point components including an ethylene dimer and a condensed solvent and a gaseous stream including uncondensed unreacted gas, and then the uncondensed unreacted gas was supplied to the compressor 10 and the condensed liquid phase was transferred to a refining column 200 again.

### Example 3:

As shown in FIG. 5, the process was performed in the same manner as in Example 1, except that in step 3, gas recovered in the separation column 200 was separately compressed in the compressor 30 in advance, and then recirculated to the reactor 100 with the compressed gas discharged from the compressor 10, and pressure in the upper portion of the reactor was directly controlled while a part of the compressed gas diverged through a pressure regulating valve 11 provided in the recirculation pipe of the compressor 10 and was recirculated to the supply line of the compressor.

### Example 4:

As shown in FIG. 6, the process was performed in the same manner as in Example 2, except that in step 3, gas recovered in the separation column 200 was separately compressed in the compressor 30 in advance, and then recirculated to the reactor 100 with the compressed gas discharged from the compressor 10, and pressure in the upper portion of the reactor was directly controlled while a part of the compressed gas diverged through a pressure regulating valve 11 provided in the recirculation pipe of the compressor 10 and was recirculated to the supply line of the compressor.

### Example 5:

The process was performed in the same manner as in Example 1, except that the compressor capacity was designed so that the flow rate of the compressed gas which diverged in the compressor discharge line 10b and was recirculated in step 3 was 10% of the total compressed gas flow rate.

### Example 6:

The process was performed in the same manner as in Example 1, except that the compressor capacity was designed so that the flow rate of the compressed gas which diverged in the compressor discharge line 10b and was recirculated in step 3 was 30% of the total compressed gas flow rate.

### Example 7:

The process was performed in the same manner as in Example 1, except that the compressor capacity was designed so that the flow rate of the compressed gas which diverged in the compressor discharge line 10b and was recirculated in step 3 was 50% of the total compressed gas flow rate.

### Example 8:

The process was performed in the same manner as in Example 1, except that the compressor capacity was designed so that the flow rate of the compressed gas which diverged in the compressor discharge line 10b and recirculated in step 3 was 3% of the total compressed gas flow rate.

Pressure fluctuations of the reactor 100 and the supply line 10a of the compressor in the comparative examples and the examples were calculated through simulation (using HYSYS DANAMICS program), and the results are shown in the following Table 1.

**[Table 1]**

| | Divergence /recircula tion of compressed gas | Divergenc e rate¹⁾ of compresse d gas | Fluctuation range of pressure in supply line of compressor during entire process | Fluctuation range of pressure inside reactor during entire process | Amount of electric energy used in compressor (KW) |
|---|---|---|---|---|---|
| Comparative Example 1 | × | - | more than ± 5 bar | more than ± 3 bar | 10.02 |
| Comparative Example 2 | × | - | more than ± 5 bar | more than ± 3 bar | 9.70 |
| Example 1 | ○ | 5% | ± 0.2 bar | ± 0.05 bar | 8.11 |
| Example 2 | ○ | 5% | ± 0.2 bar | ± 0.05 bar | 7.85 |
| Example 3 | ○ | 5% | ± 0.05 bar | ± 0.05 bar | 7.85 |
| Example 4 | ○ | 5% | ± 0.05 bar | ± 0.05 bar | 7.59 |
| Example 5 | ○ | 10% | ± 0.1 bar | ± 0.02 bar | 8.56 |
| Example 6 | ○ | 30% | ± 0.1 bar | ± 0.02 bar | 11.01 |
| Example 7 | ○ | 50% | ± 0.1 bar | ± 0.02 bar | 15.41 |
| Example 8 | ○ | 3% | ± 1.0 bar | ± 0.50 bar | 7.94 |
| 1) represents diverging and recirculated flow rate (wt%) based on the total flow rate of compressed gas. | | | | | |

It was found from Table 1 that in Comparative Examples 1 and **2,** the capacity of the compressor was designed to be larger by 30% or more than the capacity of the supply line of the compressor, without the compressor recirculation pipe, and as a result, the compressor was stopped by an interlock set for protecting the compressor as the pressure in the supply line of the compressor was decreased. Thus, the pressure in the supply line of the compressor was increased before the compressor was operated again, and as the process was repeated, the differential pressure of the pressure regulating valve positioned in the upper portion of the reactor greatly changed to cause reactor pressure hunting. In addition, as the pressure in the supply line of the compressor greatly changed, gas recovered in the separation column, which was transferred by pressure difference in the separation column, was not smoothly supplied, and the pressure in the separation column was not able to be adjusted. That is, in Comparative Examples 1 and 2, the compressor was stopped due to a difference in pressure from the supply line of the compressor, and large pressure fluctuation occurred inside the supply line of the compressor and the reactor until the compressor was operated again.

Unlike this, in Examples 1 to 8, since the unreacted ethylene gas was compressed in the compressor, and a part of the compressed gas discharged from the compressor diverged and was recirculated to the supply line of the compressor to adjust the flow rate of the recirculated compressed gas and control the pressure in the supply line of the compressor, it was confirmed that pressure change inside the reactor was small even when a change in the flow rate of the unreacted gas discharged from the reactor occurred.

In particular, in Example 6, since the divergence rate of the compressed gas was 30%, the flow rate of the supply line of the compressor was increased by 30% to be corresponded to the compressor capacities of Comparative Examples 1 and 2, but pressure fluctuation was controlled while electricity usage was not greatly increased. This is the result of maintaining constant pressure in the supply line of the compressor by adjusting the recirculation flow rate of the compressed gas which is diverged by constant opening of the pressure regulating valve provided in the compressor recirculation pipe.

Meanwhile, in Example 7, the divergence rate of the compressed gas was 50%, and though the pressure fluctuation occurring in the supply line of the compressor was sufficiently controlled, the amount of energy used was somewhat increased as compared with Example 6.

Example 8 is an example in which the flow rate of the gas discharged from the upper portion of the reactor was instantaneously increased or the flow rate of gas recovered in the upper portion of the separation column was instantaneously increased, and since the margin of the compressor capacity was small, the pressure in the supply line of the compressor was increased, which affected reactor pressure adjustment, and thus, pressure fluctuation inside the reactor was somewhat increased.

### [Description of symbols]

100: Reactor
200: Separate column
10, 30: Compressor
11: Pressure regulating valve of compressor recirculation pipe
20: Condenser

## Claims

1. Amethod of preparing an oligomer, the method comprising:
(S1) supplying an ethylene gas to a lower liquid area including a solvent in a reactor to perform an oligomerization reaction, discharging an unreacted ethylene gas to an upper portion of the reactor, and discharging a liquid stream including an oligomer product to a lower side of the reactor;
(S2) supplying the liquid stream discharged from the lower side of the reactor to a separation column, and separating gas from the liquid stream to obtain a stream including the oligomer product; and
(S3) supplying the gas discharged from the upper portion of the reactor to a compressor in which a recirculation pipe is installed to compress the gas, and then recirculating a compressed gas to the reactor,
wherein a part of the compressed gas discharged from the compressor diverges to the compressor, and the compressed gas which is diverged is recirculated to a supply line of the compressor through a pressure regulating valve provided in the recirculation pipe of the compressor to control pressure in the supply line of the compressor.

2. The method of preparing an oligomer of claim 1, wherein the gas separated in the separation column is recovered and transferred to the supply line of the compressor.

3. The method of preparing an oligomer of claim 1, wherein the gas supplied to the compressor is obtained by separating low-boiling point components and a solvent which are condensed in a condenser placed in a front stage of the compressor.

4. The method of preparing an oligomer of claim 3, wherein the low-boiling point components and the solvent which are condensed in the condenser are transferred to the separation column after the separation.

5. The method of preparing an oligomer of claim 1, wherein the pressure regulating valve provided in the recirculation pipe of the compressor remains constantly open to continuously recirculate the compressed gas which is diverged.

6. The method of preparing an oligomer of claim 1, wherein a flow rate of the compressed gas recirculated to the supply line of the compressor is 5 to 50 wt% of the total flow rate of the compressed gas discharged from the compressor.

7. The method of preparing an oligomer of claim 6, wherein the flow rate of the compressed gas recirculated to the supply line of the compressor is 10 to 30 wt% of the total flow rate of the compressed gas discharged from the compressor.

8. The method of preparing an oligomer of claim 1, wherein the gas separated in the separation column is recovered, and compressed in advance and then transferred to a discharge line of the compressor.

9. The method of preparing an oligomer of claim 1, wherein a pressure fluctuation in the supply line of the compressor is controlled within a range of -1 bar to 1 bar.

10. The method of preparing an oligomer of claim 9, wherein the pressure fluctuation in the supply line of the compressor is controlled within a range of -0.2 bar to 0.2 bar.

11. The method of preparing an oligomer of claim 1, wherein a pressure fluctuation during operation of the reactor is controlled within a range of -0.5 bar to 0.5 bar.

12. The method of preparing an oligomer of claim 11, wherein the pressure fluctuation during the operation of the reactor is controlled within a range of -0.1 bar to 0.1 bar.
